(19) Europäisches Patentamt
European Patent Office
Office européen des brevets

(11) **EP 3 815 605 A1**

(12) **EUROPEAN PATENT APPLICATION**

(43) Date of publication:
**05.05.2021 Bulletin 2021/18**

(51) Int Cl.:
*A61B 5/0507* (2021.01)    *A61B 5/00* (2006.01)
*H01P 3/00* (2006.01)

(21) Application number: **20205299.9**

(22) Date of filing: **02.11.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.10.2019  PT 2019115880**

(71) Applicant: **Universidade do Minho
4704-553 Braga (PT)**

(72) Inventors:
- **MATEUS MENDES, PAULO
 4800-058 GUIMARÃES (PT)**
- **DA SILVA CARDOSO, VERA CAROLINA
 4800-058 GUIMARÃES (PT)**
- **JMAI, BASSEM
 4800-058 GUIMARÃES (PT)**
- **DA COSTA DINIS, HUGO DANIEL
 4800-058 GUIMARÃES (PT)**

(74) Representative: **Patentree
Edificio Net
Rua de Salazares, 842
4149-002 Porto (PT)**

(54) **PROBE AND SYSTEM FOR DETECTING AND MEASURING BRAIN TUMOR CELLS, FABRICATION METHOD THEREOF**

(57)    The present disclosure relates to a probe for detecting or measuring brain tumour cells using millimetre waves, wherein the probe comprises a microwave waveguide for conducting electromagnetic waves towards the brain, said probe further comprising a readout electronic element and a signal generator; wherein the waveguide further comprises an excitation source port for the signal generator and two measurement ports for the electromagnetic waves inside the waveguide; wherein the readout electronic element comprises electromagnetic transducers for said measurement ports for measurement of an outgoing electromagnetic signal to the brain and a reflected signal from the brain; wherein the readout electronic element is placed on the waveguide. The present disclosure also relates to a system comprising the probe for detecting or measuring brain tumour cells of the present disclosure and a method of fabrication of the probe of the present disclosure.

**Fig. 5**

**Description**

**TECHNICAL FIELD**

The present disclosure relates to the detection of tumour cells. It is applied in the medicine field and provides a device to detect and/or measure brain tumour cells through the use of MMW (MilliMeter Waves - high frequency signals) technology in order to detect small tumour sizes.

**BACKGROUND**

Cancer is a major public health problem worldwide and is the second most common cause of death in Portugal with 24.3% of these deaths caused by malignant neoplasms [1]. Cancers of the brain and central nervous system tumours constitute a group of rare and heterogeneous tumours. These tumours represent approximately 3% of cancer cases worldwide and they are more frequent among men than women [2]. Despite the low incidence rate, brain cancers appear in the top 10 of cancer mortality [3], have one of the most devastating prognoses and are one of the cancers that have fewer treatments solutions [4].

When brain cancer is diagnosed, surgery is usually the best solution, and a higher resection degree of the lesion is associated with greater survival rates of the patient [3], [5]. However, until a few years, the tumour total resection was a task with a low success rate due to the similarity between the appearance of the tumour and surrounding brain parenchyma. Furthermore, most of the existing tools, such as Magnetic Resonance Imaging (MRI), fluorescent dyes, ultrasound and mass electroscopy, have limitations in several aspects when applied in brain cancer resection. In fact, errors in resection task, as extremely aggressive resections, can produce severe neurological damage, while less aggressive resection can leave significant residual diseased tissue [3]. Tumors, as malignant gliomas, do not have a distinctive margin between the tumour mass and the surrounding brain, representing a major challenge to the neurosurgeon compared with other tumours [3], [5]. This is the major problem and the reason for the poor prognosis of brain tumor patients.

Studies that examining patterns of brain tumour recurrence have repeatedly shown that 80-90% of the recurrences are due to residual tumour cells left behind due to incomplete resections [6]. Therefore, it is important the emergence of new surgical tools and methods to increase the resection quality and depth.

According to realized studies, it was noticed that human tissues have a frequency-specific dependent response. In fact, laboratory measurements have been demonstrated to detect tumors using low frequencies (1 Hz to 100 kHz), microwaves (1-10 GHz), and millimeter waves (10-30 GHz) [7], [8]. Furthermore, studies based on the dielectric properties of tissues concluded that these properties differ between tissue types [11].

Most measurements and studies were based on tissue electromagnetic properties, and most of them conclude that properties typically are different when measured in different tissue types.

In order to evaluate the response of both brain and tumour tissues, when electric signals are applied to them, the properties that must be known are their relative permittivities and conductivities.

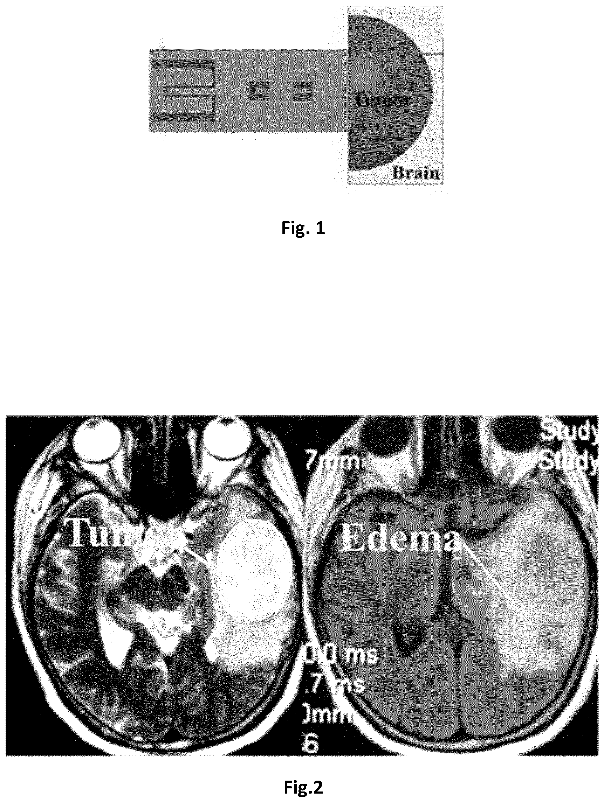

Aggressive brain tumours, metastatic tumours, malignant gliomas and any benign tumours such as meningiomas, produce a brain edema (an increased water content of the brain parenchyma) [12]. Therefore, in some cases, there are significant differences between a completely healthy brain (all the area around the edema and tumour until it reaches the bone) and a brain with a tumour, as it can be seen in Fig. 2.

A person skilled in the art knows that the brain edema can occur as a consequence of many neurological conditions, and the tissue's water content can increase from 77% to 80% [13]. In this way, if we measure in some way the result of such water content modification, we can classify the different types of tissues. For this reason, it is necessary to determine and distinguish the dielectric properties of healthy brain, tumour, and brain with edema.

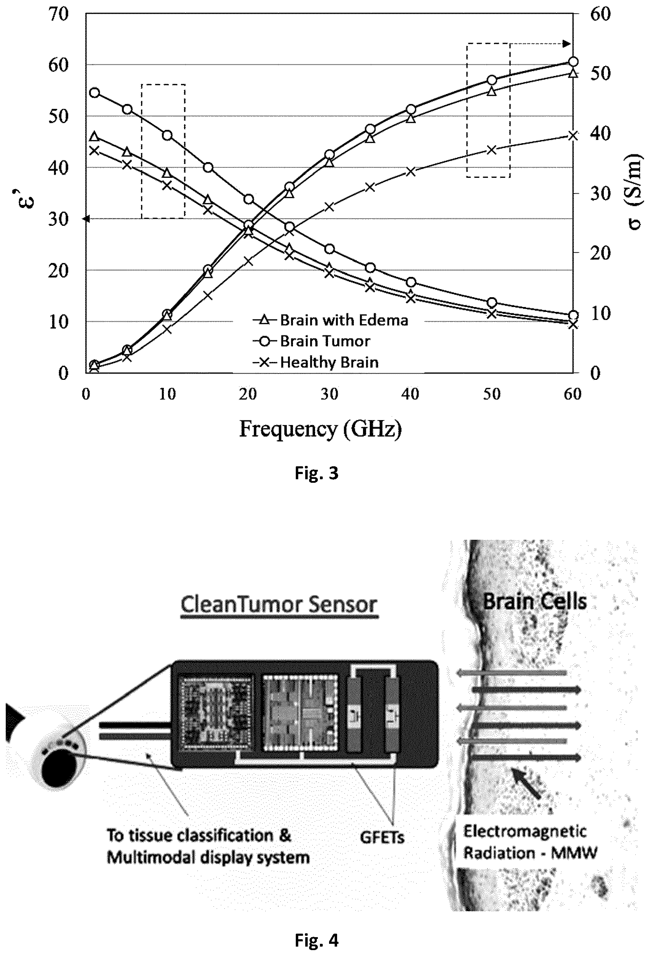

In the ultra-high frequency (UHF) range and higher, the electrical properties of a tissue are almost entirely determined by its water content due to the polarization of the water dipoles [14]. Consequently, it is possible to predict the dielectric properties of soft tissues by performing a measurement of their water content [15]. In this sense, the empirical equations presented by Schepps and Foster in [15] were duly used in the present invention to define the permittivity ($\varepsilon$') and conductivity ($\sigma$) of tumor, brain and brain with edema, as showed in Fig. 3.

The present disclosure applies the measurement of dielectric properties on human tissues using electromagnetic waves, where in one option, instead of having a cable between the probe and a measurement desktop device performing all measurements, all the relevant measurements are done on the developed probe. In this way, eliminating the cable that forces to have a calibration for each measurement, as required at millimetre wave, the system will have accuracy, repeatability, and enough sensibility to be used as a tool for medical support. It refers to the development of a probe comprising a waveguide microwave for the detection of tumor cells, where the relevant and critical measurements are made at the probe tip.

These facts are disclosed in order to illustrate the technical problem addressed by the present disclosure.

**GENERAL DESCRIPTION**

The present disclosure relates to a probe for detecting/measuring tumour cells with a resolution in the order of millimetres or smaller, to ensure that, when tumour cells are removed, the remaining tumour volumes have sub-millimetre sizes. The device has high resolution (sub-millimeter) and works based on high frequency millimeter waves (MMW), like the ones used for mobile communication systems, to support the detection as well the information acquisition and delivery. The MMW technology is integrated in the device providing a fast and precise measure technology.

A person skilled in the art will understand that sub millimeter term should be any kind of unit of measure smaller than millimeters, like as, micrometers, nanometers or any other unit of measure.

In an embodiment, the probe measures the dielectric properties on human tissues using electromagnetic waves. More particularly, uses high frequency MMW, that are guided through a waveguide, to perform tumor imaging.

In an embodiment, the probe is capable to detect tumor tissues. More particularly, the probe is able to read the differences between a signal obtained from a volume of tumoral cells and a signal obtained from non-tumoral cells.

In an embodiment, the device operates at a frequency range of 40 - 100 GHz, preferably at a frequency range of 60-100 GHz, more preferably at 60 GHz. Considering that the dielectric constant of human tissues is higher than one, together with the approach used to measure the electromagnetic properties, the probed described in the present disclosure, leads to the possibility to detect regions smaller than 1 mm thick.

The present disclosure comprises a probe that can be connected on a holder; a readout electronic element; a signal generator and an external system.

The holder might be selected from any kind of support element or a surgical tool.

The probe can be connected on the holder by whichever physical means are usually used, like as glued, fitted or other means. The probe is made of any suitable material for use with microtechnology processing, for example, silicon, quartz, silica, glass. Preferable is made of silicon.

In an embodiment, the probe may be optionally coated with aluminium, gold, silver, titanium, or any non-toxic conductive surface. For detection purposes, it may also be coated with a functionalized material like an electroactive material, a magnetoactive material, an electro fluorescent material, or a surface that binds with tumour cells, to mention a few examples.

In an embodiment, the probe comprises at least 2 ports and a readout electronic element for the MMW (high frequency signals), specifically an electromagnetic transducer. With this readout electronic element, there is no need to calibrate MMW system prior to each measurement. One port is the excitation source, region where the electromagnetic wave will be applied and the others are measurement ports - where the sensing electronics will be connected to record a signal that will allow to sense what type of tissue is in touch with the probe.

In an embodiment, the readout element might be selected from a list comprising readout transistors, magnetic sensors, electric sensors, or any transducer that can translate the variation of the electromagnetic wave inside the waveguide into a voltage, current, or charge.

In an embodiment, the signal generator is integrated within the probe.

In another embodiment, the signal generator is connected to the probe by means of a cable.

In an embodiment, the probe is for detecting and/or measuring tumour cells in the patient's brain.

The present disclosure also relates to a device comprising the probe and a holder to support said probe. In an embodiment, the probe comprises at least one excitation source and two millimetre wave signal detectors.

In an embodiment, the device is connected to an external device configured to analyze the data detected by the wave signal detectors.

In an embodiment, the external device is a computer or any other electronic mean that analyses the signal detected and received by the probe and gives information about the tissue that is being analyzed.

In an embodiment, the probe can be near or close to the tissue to be analyzed, or can reach it and be in contact with the tissue (touching). The readout electronic element will register the outgoing signal from the tissue and afterwards the reflected signal. The readout output is then changed according to the different properties of the tissue that is nearby the probe.

In an embodiment, the data obtained from the analysed tissue is processed by the external device, based on an algorithm developed to classify the condition of the tissue, i.e., if the tissue is a healthy tissue or a tumour tissue. The status of the tissue is then provided to the user (surgeon) in the form of an image, a sound, among others.

In an embodiment, the probe uses preferably the available 5G technologies that are in development in the MMW region, but any other technology providing the required frequencies can be used for this purpose.

In an embodiment, the device must be very small in order to allow the detection of regions smaller than 1 mm$^2$ and this is assured if the signal frequency is high, thus the signal wavelength is low, which is why a high frequency (MMW) of 30-100 GHz, namely 60 GHz, is used.

In another embodiment, the probe may also be applied in any other applications from different fields such as, but not limited to, measure the properties of a grape (to assess its maturity state); measure the properties of human skin, allowing to detect a skin tumour; to be applied in lab-on-chip devices to detect the properties of fluids going through its channels.

In an aspect, the present disclosure relates to a system to detect or measure the tumour cells comprising: a holder; a probe; a readout electronic element; a signal generator; and an external system.

In an embodiment, said system applies a frequency range about 30 - 100 GHz to detect or measure the tumour cells.

In another embodiment, said system applies a frequency of 60 GHz to detect or measure the tumour cells.

In an embodiment, the probe is located on the holder and has at least one excitation source and one measurement element.

In an embodiment, the probe comprises a readout electronic element.

In an embodiment, the readout element might be selected from transistors, magnetic sensors or electric sensors.

In an embodiment, the signal generator is connected to the probe by means of a cable.

In an embodiment, the signal generator is integrated on the probe.

In an embodiment, the system may use any electronic auxiliary means such as computer, tablet, television, etc.

In an embodiment, the detection methodology is selected from S parameters, or others, and allows on-probe electronics integration.

In an embodiment, an external system will run a software that will analyze the signal detected on probe by an algorithm based on signal from readout electronics.

The present disclosure also relates to a probe to detect or measure the tumour cells wherein said probe is placed on a tip of a holder or on the tip of the surgical tool, has a small dimension and uses microtechonologies to properly guide the electromagnetic wave close to the brain to detect or measure the tumour cells sizes.

In an embodiment, the probe dimension is adapted to the tumour size and frequency of interest, and the size and geometry of the probe must be adapted to the detection methodology.

In an embodiment, the dimension of said probe is, preferably with high about $10\mu$m to 10mm, thickness about $10\mu$m to 10mm and length about $10\mu$m to 10cm.

In an embodiment, said probe operates at MMW (high frequency signals).

In an embodiment, a readout electronics element is duly placed on the probe tip.

In an embodiment, said probe is connected externally to a signal generator through a cable connection which will allow that the measurement of the tumour size occurs directly in the probe.

An aspect of the present disclosure relates to a probe for detecting or measuring brain tumour cells using millimetre waves, MMW, of 30 - 100 GHz, said probe being for connecting to a probe holder, said probe for being used with an external system, said probe comprising a microwave waveguide for conducting electromagnetic waves towards the brain, said probe further comprising a readout electronic element and a signal generator; wherein the waveguide further comprises an excitation source port for the signal generator and two measurement ports for the electromagnetic waves inside the waveguide; wherein the readout electronic element comprises electromagnetic transducers for said measurement ports for measurement of an outgoing electromagnetic signal to the brain and a reflected signal from the brain; wherein the readout electronic element is placed on the waveguide.

In an embodiment for better results, the frequency is 60-100 GHz.

In an embodiment for better results, the electromagnetic transducers are readout transistors, magnetic sensors, or electric sensors, in particular graphene field effect transistors.

In an embodiment for better results, the signal generator is integrated within the probe or the signal generators is external and connected to the probe by a cable.

In an embodiment for better results, the waveguide has a height of 10 $\mu$m to 10 mm; a thickness of 10 $\mu$m to 10 mm, and a length of 10 $\mu$m to 10 cm.

In an embodiment for better results, the waveguide is obtainable by microfabrication.

In an embodiment for better results, the probe waveguide is coated with a conductive surface and is made of silicon, quartz, silica, or glass, in particular silicon, further in particular microfabrication silicon wafer.

In an embodiment for better results, the conductive surface coating is a coating of aluminium, gold, silver, titanium, and/or a coating of a functionalized material, in particularly an electroactive material, a magnetoactive material, an electro fluorescent material, or a surface that binds with tumour cells.

In an embodiment, the probe of the present disclosure may be used for detecting or measuring grape maturity, human skin properties or skin tumour, or fluid properties of fluids going through lab-on-chip channels.

In an embodiment, the probe of the present disclosure may comprise readout electronics for registering the outgoing electromagnetic signal to the brain and the reflected signal from the brain wherein the readout electronics are integrated into the probe.

Another aspect of the present disclosure relates to a system comprising the probe for detecting or measuring brain tumour cells of the present disclosure.

In an embodiment, the system may comprise a probe holder to support said probe.

In an embodiment, the system may comprise an external computer system connected to the probe and configured to analyse the registered electromagnetic signal for detecting or measuring brain tumour cells.

Another aspect of the present disclosure relates to a fabrication method for obtaining a probe according to any of the claims 1-9 comprising the steps of:

   providing a silicon wafer;
   fabricating detectors in said silicon wafer for the electromagnetic transducers;
   defining the waveguide on silicon;
   metallization of the waveguide on a front and a back of the wafer;
   interconnecting the obtained probe with a signal generator, optionally integrated on the probe.

In an embodiment, the detectors may be graphene transistors, GFETs, among ithers.

## BRIEF DESCRIPTION OF THE DRAWINGS

The following figures provide preferred embodiments for illustrating the disclosure and should not be seen as limiting the scope of invention.

Figure 1 - It is an embodiment of the device described in the present disclosure.

Figure 2 - It is the T2 MRI images of a patient with a brain tumour which produced edema on surrounding cells [13].

Figure 3 - Shows the permittivity and conductivity data from brain with edema, healthy brain and brain tumour.

Figure 4 - It is a schematic representation of the proposed probe and interaction with a brain region.

Figure 5 - It is a schematic representation of an embodiment of the waveguide model with 3 ports. H = 290 $\mu$m, L = 3000 $\mu$m and We = 1240 $\mu$m.

Figure 6 - It is a schematic representation of different tumor sizes, 1, 2, 3, 4 and 5, during a simulated resection.

Figure 7 - It is an embodiment of the results from S23 measurement. Plot A) represents the amplitude and phase difference between normal brain with different tumor sizes, and normal brain only. Plot B) represents the amplitude and phase difference between brain edema with different tumor sizes, and brain edema only.

Figure 8 - It is an embodiment of the electric field pattern and magnitude in the waveguide for two different scenarios a and b. The patterns a1, 2, 3, 4 and 5 represent the Brain + Tumor case with different tumor sizes and the patterns b1, 2, 3, 4 and 5 the Brain Edema + Tumor case also with different tumor sizes.

Figure 9 - It is an embodiment of simulated results of cases a and b, a1 and b1 without brain cells infiltration and a1.1 and b1.1 with brain cells infiltration.

Figure 10 - It is a schematic representation of an embodiment of the device to detect the tumor volume.

Figure 11 - It is an embodiment of the simulation results: difference Magnitude in S11; S12; S13 and S23.

Figure 12 - It is an embodiment of the simulation results difference in: (a) Electric Fields; (b) Magnetic Fields position 1; (c) Magnetic Fields position 2.

Figure 13 - It is a schematic representation of an embodiment of an Array of Waveguides to obtain a 2-dimensional image, 2D, image of the Tumour.

Figure 14 - It is a schematic representation of an embodiment of the fabrication method used to obtain the device, considering that graphene silicon detectors are placed in such a way that they will measure the simulated S23.

Figure 15 - It is a schematic representation of an embodiment of the fabrication steps for one probe prototype. This method does not yet include the integration of electronics.

Figure 16 - It is an example of one fabricated probe, without the integrated electronics.

Figure 17 - It is an embodiment of for the integration of the information generated by the device within a surgical environment.

**DETAILED DESCRIPTION**

The probe described in the present disclosure should be used during a surgery, like as a craniotomy, when the brain is exposed. However, other options of use are possible where the medical doctor needs to use it to decide about the volume of tumour to remove. It is just needed that the probe may be close or touching a healthy tissue, and after it will be able to detect tissues differences. If the size of tumour is high, the probe just needs to be close to the tumour to measure the size. Otherwise, when the surgeon needs more accurate information, the probe should touch the tumour to measure the size. The figure 4 shows an embodiment of the probe, and how it may interact with the brain during the surgery.

In an embodiment, the probe records the properties of the tissue to be analyzed when it reaches the vicinities or touches said tissue. Therefore, the probe should be moved in order to analyze different regions of the tissue. A 2D image of the tissue can then be retrieved.

In general lines, the surgeon approaches/touches the brain with the probe and the brain tissue properties of that patient are recorded. After, during the procedure, the surgeon approaches/touches the region affected by the tumour and a display, based on the signal collected by the probe, gives the information about the properties of the analyzed tissue, namely the volume of healthy and tumour regions.

Depending on the difference between healthy tissue values and the values registered at each moment, the surgeon may decide about what to do. Remove more tumour tissue or finishing the procedure.

In an embodiment, the probe's waveguide is fabricated in a small dimension using microtechnologies to properly guide the electromagnetic wave close to the brain, and receive the reflected signal from the brain. The use of microtechnologies allows to obtain the waveguide with a size in the order of the tumour sizes to be investigated.

In an embodiment, the probe may have any dimension allowed by the microfabrication processes, but preferably it must be millimeters sized, or sub-millimeters, for example, dimensions selected between high about 10 $\mu$m to 10 mm, thickness about 10 $\mu$m to 10 mm, and length about 10 $\mu$m to 10 cm, but not limitative to this dimension. However, the dimension must be adapted to the frequency of interest, which is previously selected in accordance with the minimum dimension value of the tumour of interest. Also, the size and geometry of the probe must be adapted to the detection methodology. The detection methodology should be selected from S parameters, or others, and should allow on-probe electronics integration.

In an embodiment, the probe's waveguide, is preferably made of silicon or by any other material that can be tailored to the small required dimensions, with the required precision, and available for processing with microtechnologies, and at the same time, must allow the integration of the electronic readout mechanism on the probe. Said probe can be placed on a tip of a holder or on the tip of a surgical tool, such as a canula for tumour extraction. The probe operates at MMW in order to be able to detect small tumour sizes (in the mm range, or below).

In an embodiment for an accurate result, the probe should be placed near or in touch with the tissue of interest, such as in the brain, or in any other place where the surgeon can touch the tissue of interest. The probe should be used for any brain region that the surgeon has access during the surgery.

Since the frequencies are very high, the disclosure includes the use of the probe on an intra-operatory room integrated with the readout electronics element into the probe. Specifically, the readout element is duly placed on the probe tip. This mechanism allows skipping the step of electronic calibration of the MMW system prior to each measurement, that would not be feasible in an intra-operatory scenario since each time the probe changes the position, the path between reader and probe would change and that would require new calibration. The readout electronics element registers the outgoing signal to the brain and the reflected signal, or combinations of both. It then responds, and optionally changes the output derived from different properties of the tissue nearby the probe.

In an embodiment, the probe also requires a signal generator element. Since the measurement occurs directly in the probe, the signal generator may be placed externally and connected with a cable connection. In another embodiment, the signal generator is placed on the probe tip using preferable microtechnologies that allow the electronic integration with the probe.

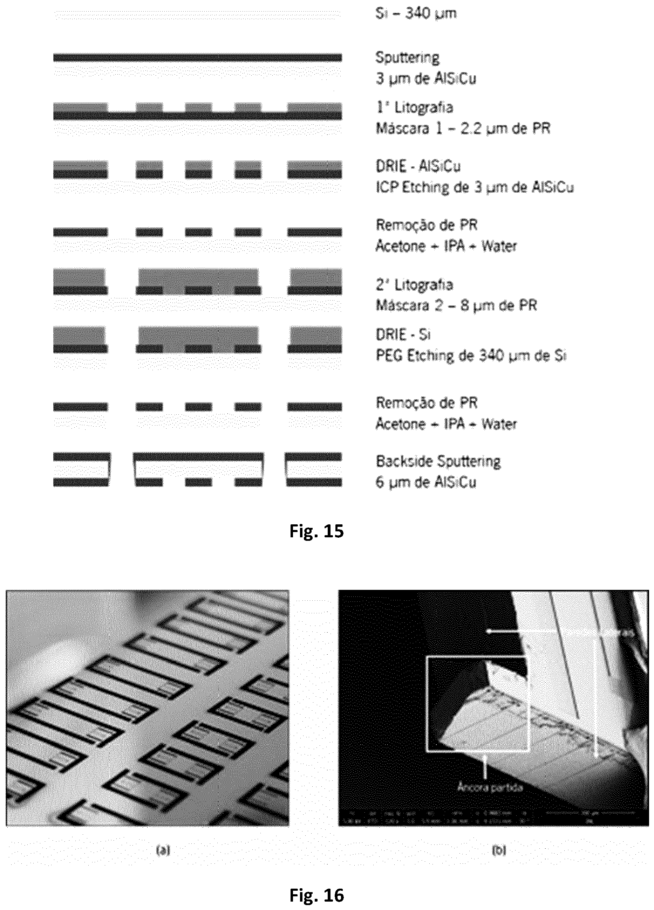

In an embodiment, an external system, like a computer, tablet, television or any other equipment for this purpose, is placed for example in the surgery room, and runs a software that analyzes the signal detected by the probe, using an algorithm that, based on the signal from readout electronics, gives an information about the properties of the tissue that the probe is detecting/touching, together with other image modalities or only with the tumour information calculated by the algorithm. The information can be showed in an external device (fig. 16) that present on a computer or on a 3D virtual system display.

The following pertains to an application example. In the present example, the probe comprises one main port, for the signal generator, and two auxiliary ports, to detect the properties of the tissue touching the probe. This configuration can mimic a desired real environment setup, where the signal readout can be achieved without a standard calibration, required for systems using millimeter waves. In this way, a cheaper and simple signal source can be used, avoiding the expensive, cumbersome and time-consuming process of calibration, something that would be unacceptable in a clinical process.

As proof-of-concept to perform tumour imaging, the developed probe was used on a liquid brain phantom. Other applications requiring electromagnetic imaging can also use a device comprising the probe.

In this particular example, the waveguide of the probe received the energy of an electromagnetic signal to detect the existence of tumour in the brain. The signal may be delivered to the probe by a cable attached to an external signal generator, or it may be generated on the probe itself. Also, other mechanisms can be used to transfer the energy and wirelessly power such a device (such as ultrasound, low frequency, optical, magnetic signals, among others).

The following pertains to a waveguide design. A rectangular silicon waveguide was obtained with a cross section of 1240 $\mu$m $\times$ 290 $\mu$m, in order to work at 60 GHz. The waveguide is responsible to conduct the electromagnetic signal towards the brain regions that are analyzed.

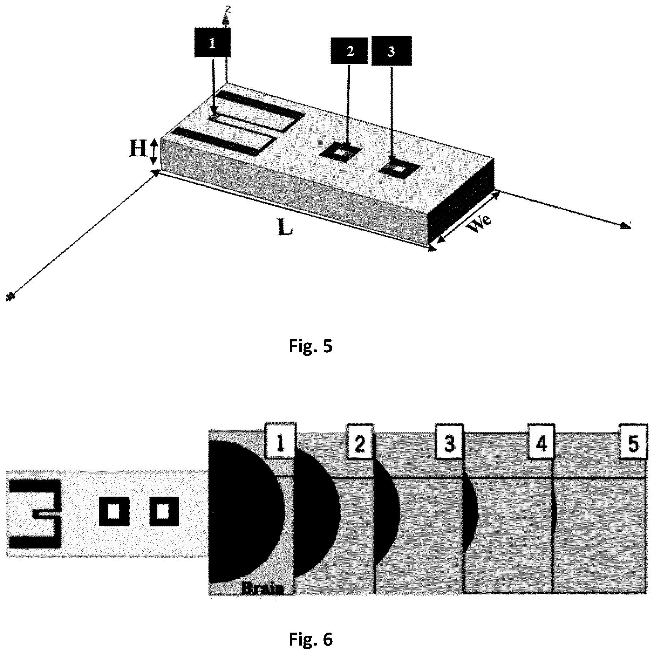

An embodiment of the rectangular waveguide is shown in Fig.5. Port 1 is the excitation source (i.e., the region where the electromagnetic wave will be applied), and ports 2 and 3 are the measurement ports, i.e., the place where the sensing electronics will be connected to record a signal that will allow to sense what type of tissue is in touch with the probe. In port 1, it is applied a 60 GHz signal, and in ports 2 and 3 it is measured the S23 parameter. In this example, two auxiliary ports were used to detect the properties of the tissue touching the probe.

The S23 parameter refers the response at port 2 due to a signal injected at port 3.

For the simulations, several surgical scenarios were modelled in order to represent the multiple possibilities that a surgeon can encounter. Firstly, different tumor sizes were studied in order to understand the expected discrimination limits from the surrounding brain parenchyma, as demonstrated in Fig. 6. It was also simulated the case of a tumour infiltrated with brain cells.

The following pertains to the measurement of S-Parameters. The measurement of S-parameters, i.e., the S23's module and phase, was the first detection method to be tested.

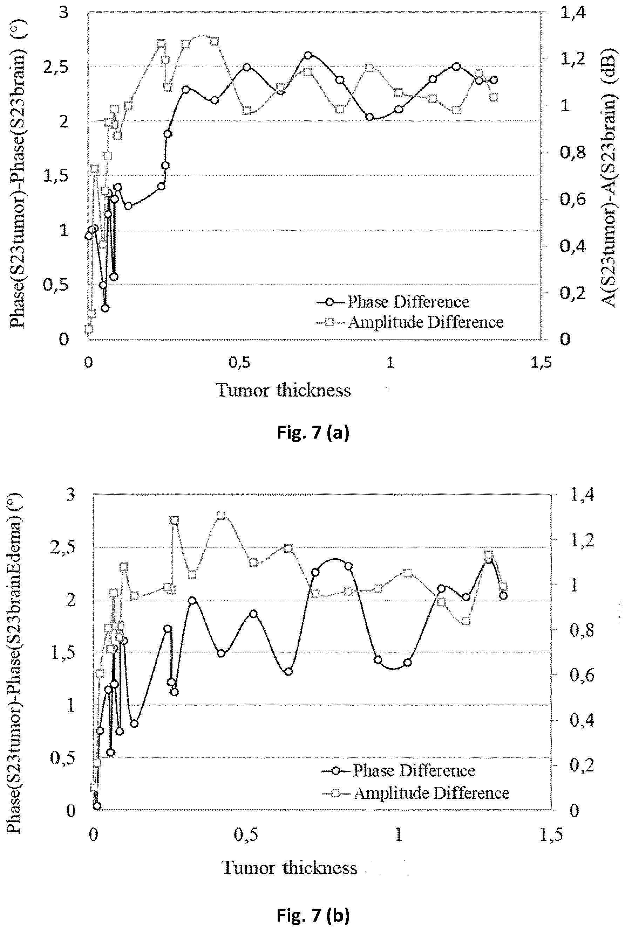

In Fig. 7, it can be seen an embodiment of the S23 amplitude and phase difference in two different scenarios, A and B, wherein scenario A uses a normal brain model and scenario B uses a brain edema models. Since a difference is observed between the tumour and non-tumour cases for both scenarios A and B, it is possible to conclude that it is conceivable to differentiate the existence of different tumour sizes in the brain parenchyma. However, the difference is small and irregular, which can be a problem in a real-life experiment due to the instability of the measurement procedure of the S-parameters and the resolution of the measurement system. Also, when an infiltration of brain cells occurs in the tumour, the difference observed in amplitude and phase is very small (Table I), which can likewise culminate in bad results.

Table I. S23 results of tumour phantom analysis, with and without brain cells infiltration.

| Brain Type | S23 | Without infiltration | With infiltration | Difference |
|---|---|---|---|---|
| Normal | A(dB) | -23.18 | -22.78 | 0.4 |
| | Phase(°) | -143.80 | -146.09 | 2.29 |
| Edema | A(dB) | -23.20 | -22.88 | 0.32 |
| | Phase(°) | -143.85 | -145.70 | 1.85 |

In another embodiment, the evaluation of the electric field distribution inside the waveguide can be used as a more accurate measurement technique as compared to S-parameters.

The following pertains to evaluation of electric field. For this experiment, it was necessary to obtain the electric field pattern in several scenarios with different tumour sizes and different types of tissues.

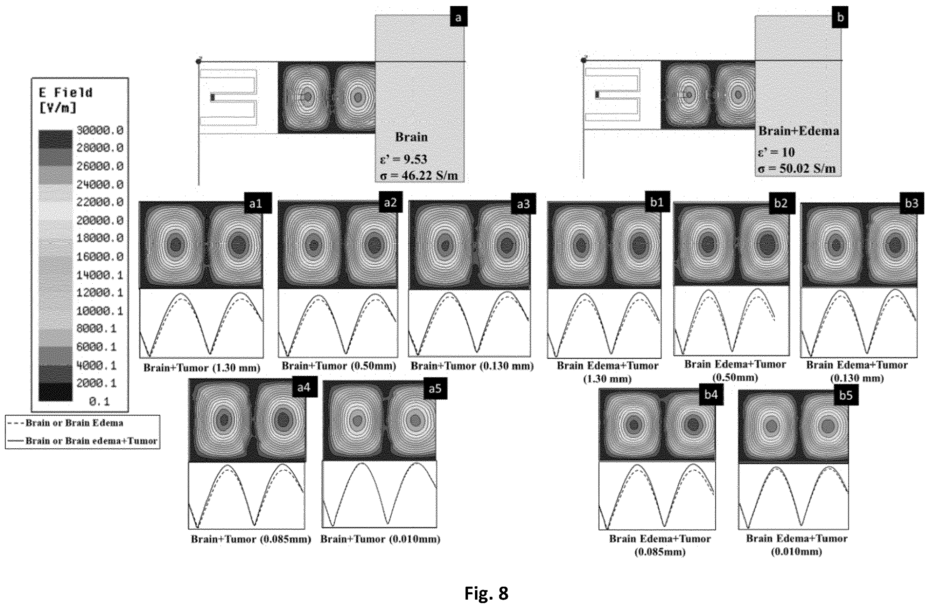

In Fig.8 it can be seen an embodiment of the simulation results of two different cases, *a* and *b*, where *a* represents the case of a brain without brain cells edema, and b the case of brain with brain cells edema. It can also be seen the electric field pattern and magnitude of a1-5 and b1-5 cases, which represent the results in presence of a tumour. The different tumor size models that were simulated can be seen in Fig.6.

Analyzing Fig. 8 it is possible to see that there are differences, approximately 13% in amplitude and 3.3% in phase, between the case when only brain was measured (a and b), and the case where the presence of a tumour inside the brain is registered (a1-5 and b1-5). On the other hand, with the decrease in tumour size, the electric field pattern starts to be the same as the one in the brain without tumour scenario. With these results, it can be concluded that it is feasible to use the evaluation of electric field to distinguish tumour from the surrounding tissue more accurately.

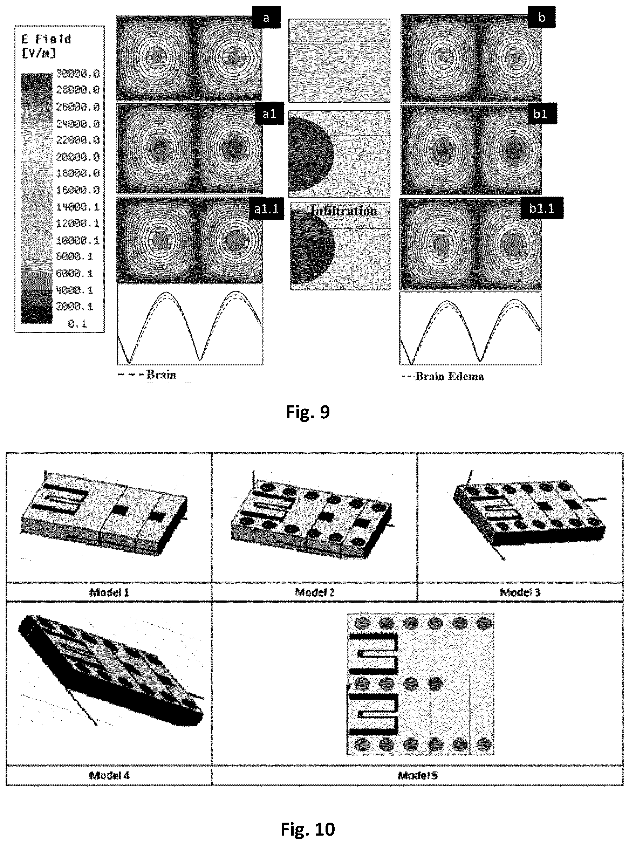

Similarly, in case of infiltration (cases a1.1 and b1.1 in Fig. 9) the results show that the electric field pattern is very similar but not equal to the brain case (case a and b in Fig. 9), as it can be seen in the magnitude plot. The electrical field pattern is also slightly different from the cases without infiltration (case a1 and b1), which indicates that there are brain cells near to the waveguide. This conclusion is important because it allows to determine that not only tumour cells exist near to waveguide but also brain cells, which is a scenario likely to occur during tumour resection.

The following pertains to other proposed models. Other examples can be presented, based on Substrate integrated waveguide (SIW) technologies and a mix between the coplanar waveguide technology (CPW) and Substrate integrated waveguide (SIW) to have a better waveguide. Figure 10 shows some types of Waveguide available. However, the present disclosure is not limited to these models of technology.

The following pertains to simulation Results. Difference of parameters was tested in where there is a tumour or not in the brain (equation. 1).

$$E_r = | S_B - S_{BT} | \qquad \text{equation. 1}$$

Where: Er is the Error, $S_B$ is the simulation of the brain intact and $S_{BT}$ is the simulation of the brain with tumour

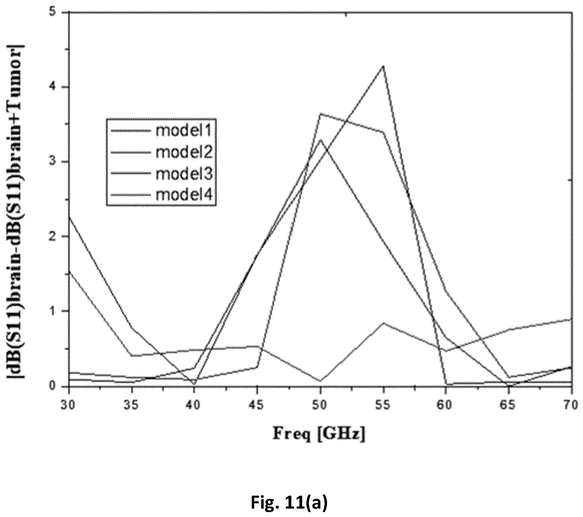

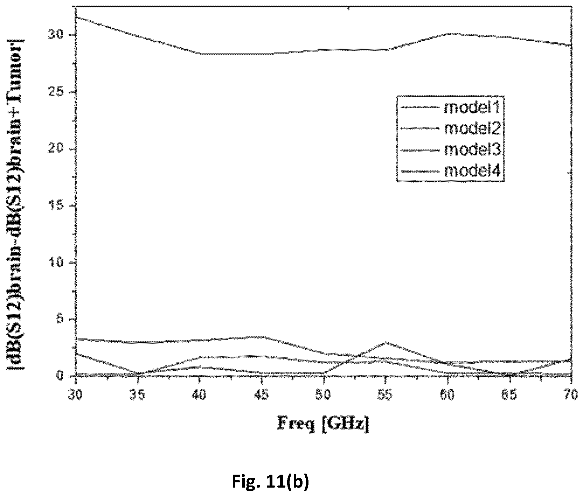

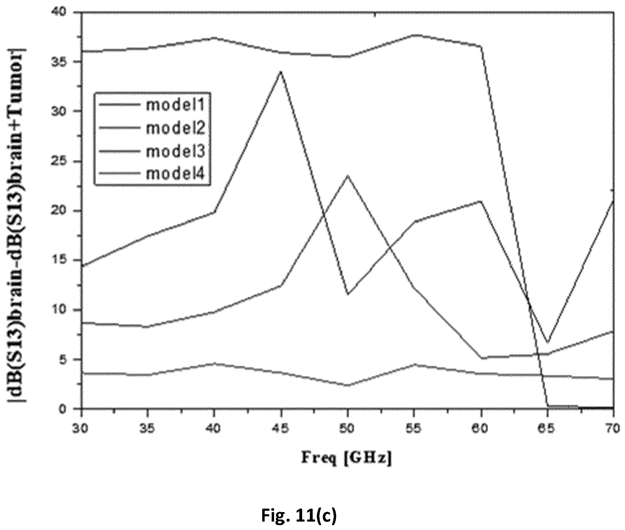

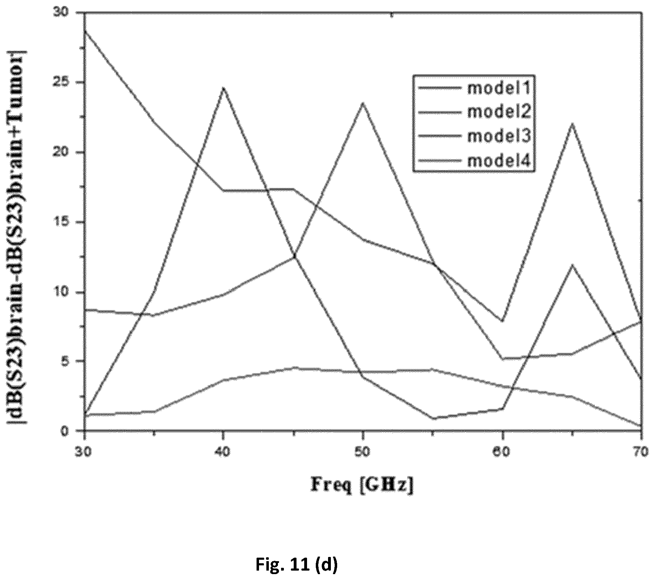

Figure 11 shows the difference magnitude between the brain intact and the simulation of the brain has tumour.

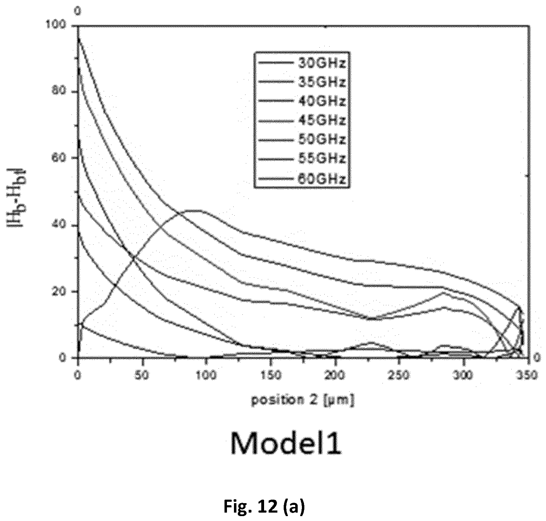

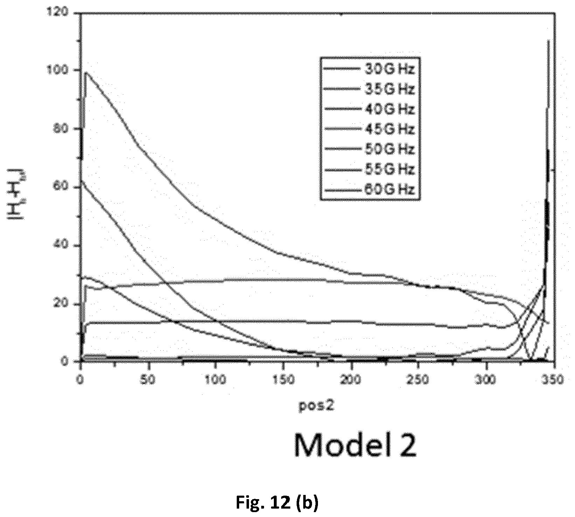

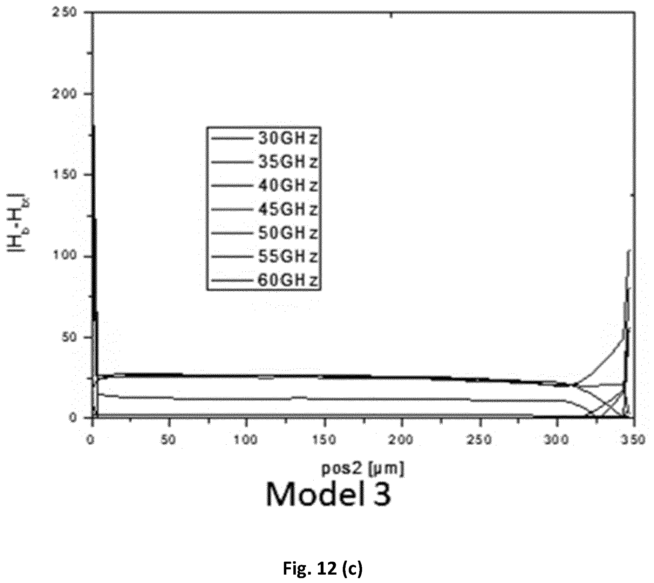

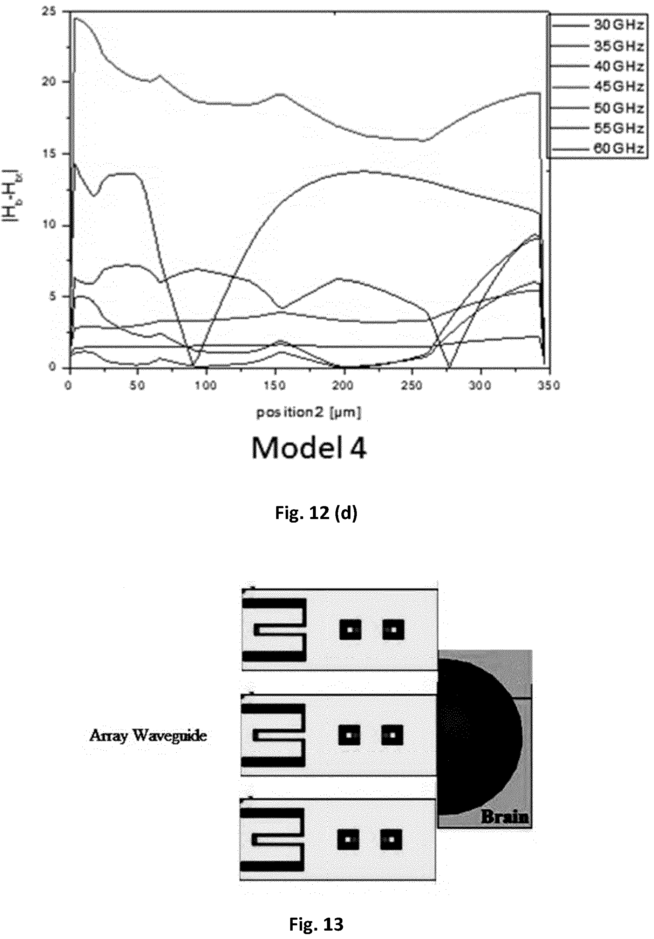

In another embodiment of simulation, it was identified the existence of tumour. Figure 12 shows the comparative study of the electric (12.a) and magnetic fields (12.b and 12.c).

The following pertains to array waveguide analysis. Figure 13 shows an embodiment of an array waveguide to identify the geometry and the size of tumour. In another embodiment, it is used a SIW Waveguide with multicavity to identify the geometry and the size of tumour.

The following pertains to detection methodology. In an embodiment, it is possible to have a modification of an electromagnetic field due to the presence of a tumour, and that modification may be able to allow the detection of small volumes of tumours, smaller than allowed by the available technologies nowadays.

In an embodiment, for validation purposes, it was used the simulation of S-parameters, being the most straightforward way of showing that the ability of a microprobe may have to detect small tumours, when it uses a high frequency signal, such as millimeter wave (MMW) signals.

In another embodiment, it is possible to use other methods (lumped or distributed) to register and translate the interaction between the tumour and probe to a readout signal. Instead of measuring only two-port S-parameters, the probe may be coated with other sensing mechanism, like readout transistors, magnetic sensors, electric sensors. In yet another embodiment, the probe surface can be coated with functionalized materials (e.g., piezolectric, magnetoelectric, electro fluorescent, or surfaces functionalized to bind with tumor cells) that interact with the tumor, changing such material properties and leading to a readout by a MMW signal, or by an optical signal, or low frequency electrical signal.

In an embodiment, the excitation signal can be generated by any kind of controllable signal or wave, like as optical, electrical, acoustic, electromagnetic signal (RF, MMW, THz).

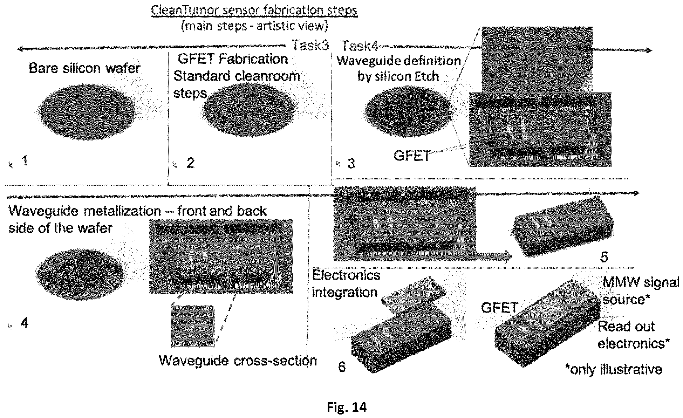

Figure 14 shows an embodiment for the probe fabrication, and integration with readout electronics. This is an illustrative scheme to show how the fabrication can be and how it can be integrated with nanotransistors. Other solutions, as presented in the simulations section, are also possible.

In an embodiment, the fabrication starts with a silicon wafer (1), where it is fabricated the detectors (in this scenario, graphene transistors - GFET) (2), after the waveguides are defined on silicon (2). The obtained structure is then finalized with a metal coating (4). After, the detachment of the probe from the initial silicon wafer (1), the detection probe (6) can be interconnected with a signal generator (5). Optionally, a signal generator can be integrated on the probe (6).

Figure 15 shows an embodiment of the implementation of the steps 3, 4, and 5 of figure 14.

The figure 16 shows an embodiment of the microprobe fabricated using microtechology.

These fabrications methods are shown to demonstrate the feasibility of such probe and are by no ways limitative to fabricate the probe using other kind of fabrication methods or steps.

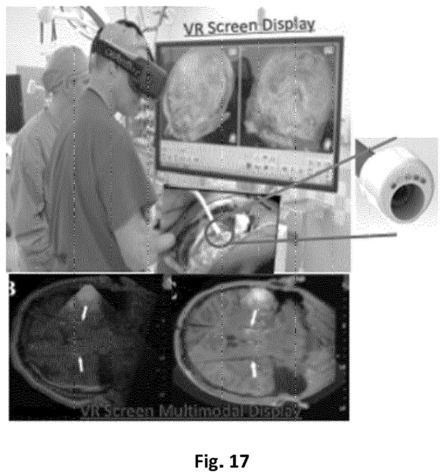

The following pertains to a full operating device. The present description discloses the core technology of the proposed sensing methodology. Figure 17 shows an embodiment of the use of such device on a real scenario. In this figure, it is presented an example on how the probe would look like and how it would be integrated in the surgical environment. In an embodiment, the signal acquired by the probe is be collected and fed to the imaging system (MRI, Ultrasound, Positron emission tomography (PET), or other used by the surgeon during or before the surgery) that is used as surgery auxiliary. As example, presurgical MRI images can be fused with real time ultrasound images, that are fed in real time to a virtual reality device that merges the images with optical images from a microscope to give a 3D image of surgical area.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

References:

[1] J. M. Lopes, F. R. Gonçalves, M. Borges, P. Redondo, and J. Laranja-Pontes, "The cost of cancer treatment in Portugal," Ecancermedicalscience, vol. 11, pp. 1-10, 2017.

[2] I. Deltour, M. Piñeros, A. Miranda-Filho, F. Bray, and I. Soerjomataram, "Cancers of the brain and CNS: global patterns and trends in incidence," Neuro. Oncol., vol. 19, no. August 2016, p. now166, 2016.

[3] P. Garcia-Corrochano et al., "Resección microquirúrgica de glioblastoma guiada con fluoresceína intraoperatoria: evaluacion retrospectiva," Rev. Peru. Med. Exp. Salud Publica, vol. 32, no. 3, p. 471, Sep. 2015.

[4] A. B. Mariotto, K. Robin Yabroff, Y. Shao, E. J. Feuer, and M. L. Brown, "Projections of the cost of cancer care in the United States: 2010-2020," J. Natl. Cancer Inst., vol. 103, no. 2, pp. 117-128, 2011.

[5] P. Astrocytoma, D. Astrocytoma, A. Astrocytoma, G. Multiforme, A. Oligodendroglioma, and A. Ependymoma, "Brain Tumors : an Introduction," 2018.

[6] S. Zhao et al., "Intraoperative fluorescence-guided resection of high-grade malignant gliomas using 5-aminolevulinic acid-induced porphyrins: a systematic review and meta-analysis of prospective studies.," PLoS One, vol. 8, no. 5, p. e63682, May 2013.

[7] K. P. M. Guiot, "Failure pattern following complete resection plus radiotherapy and temozolomide is at the resection margin in patients with glioblastoma," no. June 2011, pp. 19-23, 2013.

[8] I. J. Bahl, S. S. Stuchly, and M. A. Stuchly, "A Microstrip Antenna for Medical Applications," MTT-S Int. Microw. Symp. Dig., vol. 80, no. May, pp. 358-360, 1980.

[9] C. Gabriel et al., "measurement of the Dielectric Properties of Tissues at Microwave Frequencies," Phys. Med. Biol., vol. 41, no. 11, pp. 2251-2269, 1996.

[10] C. Gabriel, S. Gabriel, and E. Corthout, "The dielectric properties of biological tissues: I. Literature survey," Phys. Med. Biol., vol. 41, no. 11, pp. 2231-2249, 1996.

[11] M. C. Papadopoulos, S. Saadoun, D. K. Binder, G. T. Manley, S. Krishna, and A. S. Verkman, "Molecular mechanisms of brain tumor edema," Neuroscience, vol. 129, no. 4, pp. 1011-1020, 2004.

[12] Y. Esquenazi, V. P. Lo, and K. Lee, "Critical Care Management of Cerebral Edema in Brain Tumors," J. Intensive Care Med., vol. 32, no. 1, pp. 15-24, 2017.

[13] R. F. Keep, Y. Hua, and G. Xi, "Brain Water Content: A Misunderstood Measurement?," Transl. Stroke Res., vol. 3, no. 2, pp. 263-265, 2012.

[14] E. Michel, D. Hernandez, and S. Y. Lee, "Electrical conductivity and permittivity maps of brain tissues derived from water content based on T1-weighted acquisition," Magn. Reson. Med., vol. 77, no. 3, pp. 1094-1103, 2017.

[15] J. L. Schepps and K. R. Foster, "The UHF and microwave dielectric properties of normal and tumour tissues: Variation in dielectric properties with tissue water content," Phys. Med. Biol., vol. 25, no. 6, pp. 1149-1159, 1980.

[16] G. Gentile, "Mm-wave passive components for integrated phased array antennas," 2016.

**Claims**

1. Probe for detecting or measuring brain tumour cells using millimetre waves, MMW, of 30 - 100 GHz, said probe being for connecting to a probe holder, said probe for being used with an external system, said probe comprising a microwave waveguide for conducting electromagnetic waves towards the brain,
said probe further comprising a readout electronic element and a signal generator; wherein the waveguide further comprises an excitation source port for the signal generator and two measurement ports for the electromagnetic waves inside the waveguide;
wherein the readout electronic element comprises electromagnetic transducers for said measurement ports for measurement of an outgoing electromagnetic signal to the brain and a reflected signal from the brain;
wherein the readout electronic element is placed on the waveguide.

2. Probe according to any of the previous claims wherein the frequency is 60-100 GHz.

3. Probe according to any of the previous claims wherein the electromagnetic transducers are readout transistors, magnetic sensors, or electric sensors, in particular graphene field effect transistors.

4. Probe according to any of the previous claims wherein the readout electronic element, comprising electromagnetic transducers, is arranged to measure electric field.

5. Probe according to any of the previous claims wherein the signal generator is integrated within the probe or the signal generators is external and connected to the probe by a cable.

6. Probe according to any of the previous claims wherein the waveguide has a height of 10 $\mu$m to 10 mm; a thickness of 10 $\mu$m to 10 mm, and a length of 10 $\mu$m to 10 cm.

7. Probe according to any of the previous claims wherein the waveguide is obtainable by microfabrication.

8. Probe according to any of the previous claims wherein the probe waveguide is coated with a conductive surface and is made of silicon, quartz, silica, or glass, in particular silicon, further in particular microfabrication silicon wafer.

9. Probe according to the previous claim wherein the conductive surface coating is a coating of aluminium, gold, silver, titanium, and/or a coating of a functionalized material, in particularly an electroactive material, a magnetoactive material, an electro fluorescent material, or a surface that binds with tumour cells.

10. Probe according to any of the previous claims for detecting or measuring grape maturity, human skin properties or skin tumour, or fluid properties of fluids going through lab-on-chip channels.

11. Probe according to any of the previous claims comprising readout electronics for registering the outgoing electromagnetic signal to the brain and the reflected signal from the brain wherein the readout electronics are integrated into the probe.

12. System comprising the probe according to any of the previous claims and comprising said probe holder to support said probe.

13. System according to the previous claim comprising an external computer system connected to the probe and configured to analyse the registered electromagnetic signal for detecting or measuring brain tumour cells.

14. Fabrication method for obtaining a probe according to any of the claims 1-11 comprising the steps of:

    providing a silicon wafer;
    fabricating detectors in said silicon wafer for the electromagnetic transducers;
    defining the waveguide on silicon;
    metallization of the waveguide on a front and a back of the wafer;
    interconnecting the obtained probe with a signal generator, optionally integrated on the probe.

**15.** Fabrication method according to the previous claim wherein the detectors are graphene transistors, GFETs.

**Fig. 1**

**Fig.2**

Fig. 3

Fig. 4

Fig. 5

Fig. 6

Fig. 7 (a)

Fig. 7 (b)

Fig. 8

**Fig. 9**

**Fig. 10**

**Fig. 11(a)**

**Fig. 11(b)**

Fig. 11(c)

**Fig. 11 (d)**

Model1

Fig. 12 (a)

Model 2

**Fig. 12 (b)**

Model 3

**Fig. 12 (c)**

Model 4

**Fig. 12 (d)**

Array Waveguide

Brain

**Fig. 13**

Fig. 14

| | |
|---|---|
| | Si – 340 µm |
| | Sputtering<br>3 µm de AlSiCu |
| | 1ª Litografia<br>Máscara 1 – 2.2 µm de PR |
| | DRIE - AlSiCu<br>ICP Etching de 3 µm de AlSiCu |
| | Remoção de PR<br>Acetone + IPA + Water |
| | 2ª Litografia<br>Máscara 2 – 8 µm de PR |
| | DRIE - Si<br>PEG Etching de 340 µm de Si |
| | Remoção de PR<br>Acetone + IPA + Water |
| | Backside Sputtering<br>6 µm de AlSiCu |

**Fig. 15**

(a)

(b)

Âncora partida

**Fig. 16**

**Fig. 17**

Europäisches
Patentamt

European
Patent Office

Office européen
des brevets

# EUROPEAN SEARCH REPORT

Application Number

EP 20 20 5299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NASR ISMAIL ET AL: "Single- and Dual-Port 50-100-GHz Integrated Vector Network Analyzers With On-Chip Dielectric Sensors", IEEE TRANSACTIONS ON MICROWAVE THEORY AND TECHNIQUES, PLENUM, USA, vol. 62, no. 9, 1 September 2014 (2014-09-01), pages 2168-2179, XP011557650, ISSN: 0018-9480, DOI: 10.1109/TMTT.2014.2337264 [retrieved on 2014-08-29] * abstract * * page 2168 * * page 2170 * * page 2174 - page 2177 * * figures 2,12b * | 1-15 | INV. A61B5/0507 A61B5/00 H01P3/00 |
| Y | BENJAMIN LAEMMLE ET AL: "An integrated 125GHz Sensor with read-out circuit for permittivity measurement of liquids", MICROWAVE SYMPOSIUM DIGEST (MTT), 2012 IEEE MTT-S INTERNATIONAL, IEEE, 17 June 2012 (2012-06-17), pages 1-3, XP032216828, DOI: 10.1109/MWSYM.2012.6258392 ISBN: 978-1-4673-1085-7 * abstract * * page 1 - page 2 * * figure 4 * | 1-15 | TECHNICAL FIELDS SEARCHED (IPC) A61B H01P |

-/--

The present search report has been drawn up for all claims

| Place of search | Date of completion of the search | Examiner |
|---|---|---|
| The Hague | 8 March 2021 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 20 20 5299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NAGAE SEKO ET AL: "Proposal Of Three-Port Dielectric Waveguide Probes For Human Blood Glucose Monitoring", IGARSS 2019 - 2019 IEEE INTERNATIONAL GEOSCIENCE AND REMOTE SENSING SYMPOSIUM, IEEE, 28 July 2019 (2019-07-28), pages 9188-9191, XP033655266, DOI: 10.1109/IGARSS.2019.8897800 [retrieved on 2019-11-13] * abstract * * page 9188 - page 9189 * * figure 1 * | 1-15 | |
| A | WANG DEFU ET AL: "Integrated 240-GHz Dielectric Sensor With dc Readout Circuit in a 130-nm SiGe BiCMOS Technology", IEEE TRANSACTIONS ON MICROWAVE THEORY AND TECHNIQUES, PLENUM, USA, vol. 66, no. 9, 1 September 2018 (2018-09-01), pages 4232-4241, XP011689775, ISSN: 0018-9480, DOI: 10.1109/TMTT.2018.2839104 [retrieved on 2018-09-03] * the whole document * | 1-15 | TECHNICAL FIELDS SEARCHED (IPC) |
| A | CARDOSO V ET AL: "Brain tumor modeling and resection limits using millimeter wavelengths", 2019 IEEE 6TH PORTUGUESE MEETING ON BIOENGINEERING (ENBENG), IEEE, 22 February 2019 (2019-02-22), pages 1-4, XP033539374, DOI: 10.1109/ENBENG.2019.8692552 [retrieved on 2019-04-15] * the whole document * | 1-15 | |

The present search report has been drawn up for all claims

| Place of search | Date of completion of the search | Examiner |
|---|---|---|
| The Hague | 8 March 2021 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **J. M. LOPES ; F. R. GONÇALVES ; M. BORGES ; P. REDONDO ; J. LARANJA-PONTES.** The cost of cancer treatment in Portugal. *Ecancermedicalscience,* 2017, vol. 11, 1-10
- **I. DELTOUR ; M. PIÑEROS ; A. MIRANDA-FILHO ; F. BRAY ; I. SOERJOMATARAM.** Cancers of the brain and CNS: global patterns and trends in incidence. *Neuro. Oncol.,* August 2016, vol. 19, now166
- **P. GARCIA-CORROCHANO et al.** Resección microquirúrgica de glioblastoma guiada con fluoresceína intraoperatoria: evaluacion retrospectiva. *Rev. Peru. Med. Exp. Salud Publica,* September 2015, vol. 32 (3), 471
- **A. B. MARIOTTO ; K. ROBIN YABROFF ; Y. SHAO ; E. J. FEUER ; M. L. BROWN.** Projections of the cost of cancer care in the United States: 2010-2020. *J. Natl. Cancer Inst.,* 2011, vol. 103 (2), 117-128
- **P. ASTROCYTOMA ; D. ASTROCYTOMA ; A. ASTROCYTOMA ; G. MULTIFORME ; A. OLIGODENDROGLIOMA ; A. EPENDYMOMA.** *Brain Tumors : an Introduction,* 2018
- **S. ZHAO et al.** Intraoperative fluorescence-guided resection of high-grade malignant gliomas using 5-aminolevulinic acid-induced porphyrins: a systematic review and meta-analysis of prospective studies. *PLoS One,* May 2013, vol. 8 (5), e63682
- **K. P. M. GUIOT.** *Failure pattern following complete resection plus radiotherapy and temozolomide is at the resection margin in patients with glioblastoma,* 2013, 19-23

- **I. J. BAHL ; S. S. STUCHLY ; M. A. STUCHLY.** A Microstrip Antenna for Medical Applications. *MTT-S Int. Microw. Symp. Dig.,* 1980, vol. 80, 358-360
- **C. GABRIEL et al.** measurement of the Dielectric Properties of Tissues at Microwave Frequencies. *Phys. Med. Biol.,* 1996, vol. 41 (11), 2251-2269
- **C. GABRIEL ; S. GABRIEL ; E. CORTHOUT.** The dielectric properties of biological tissues: I. Literature survey. *Phys. Med. Biol.,* 1996, vol. 41 (11), 2231-2249
- **M. C. PAPADOPOULOS ; S. SAADOUN ; D. K. BINDER ; G. T. MANLEY ; S. KRISHNA ; A. S. VERKMAN.** Molecular mechanisms of brain tumor edema. *Neuroscience,* 2004, vol. 129 (4), 1011-1020
- **Y. ESQUENAZI ; V. P. LO ; K. LEE.** Critical Care Management of Cerebral Edema in Brain Tumors. *J. Intensive Care Med.,* 2017, vol. 32 (1), 15-24
- **R. F. KEEP ; Y. HUA ; G. XI.** Brain Water Content: A Misunderstood Measurement?. *Transl. Stroke Res.,* 2012, vol. 3 (2), 263-265
- **E. MICHEL ; D. HERNANDEZ ; S. Y. LEE.** Electrical conductivity and permittivity maps of brain tissues derived from water content based on T1-weighted acquisition. *Magn. Reson. Med.,* 2017, vol. 77 (3), 1094-1103
- **J. L. SCHEPPS ; K. R. FOSTER.** he UHF and microwave dielectric properties of normal and tumour tissues: Variation in dielectric properties with tissue water content. *Phys. Med. Biol.,* 1980, vol. 25 (6), 1149-1159
- **G. GENTILE.** *Mm-wave passive components for integrated phased array antennas,* 2016